# EUROPEAN PATENT SPECIFICATION

(11) **EP 0 355 425 B1**
(45) Date of publication and mention of the grant of the patent: **22.09.1993**
(21) Application number: 89113487.6
(22) Date of filing: 22.07.1989
(51) Int. Cl.: B65B 55/16

(54) **Method and apparatus for microwave sterilization of ampules**
Verfahren und Vorrichtung zum Sterilisieren von Ampullen durch Mikrowellen
Procédé et appareil pour stériliser des ampoules pour micro-ondes

(30) Priority: 29.07.1988 JP 189880/88
(43) Date of publication of application: 28.02.1990
(73) Proprietor: Eisai Co., Ltd., Tokyo (JP); MICRO DENSHI CO. LTD., Niiza-shi Saitama-pref. (JP)
(72) Inventor: Iijima, Kenichi, Isezaki-shi Gunma-ken (JP); Kudo, Minoru, Hiki-gun Saitama-ken (JP)
(74) Representative: Hoffmeister, Helmut, Dr. Dipl.-Phys.

(56) References cited:
- FR-A- 2 166 973
- FR-A- 2 247 388
- US-A- 3 674 422
- US-A- 3 880 586

## Description

### BACKGROUND OF THE INVENTION

The present invention relates to method and apparatus far heat sterilization of sealed ampules billed with medical fluid and moving along a slot formed in upper wall of an irradiation furnace provided in communication with a rectangular waveguide by microwave irradiation occurring within said irradiation furnace.

The sealed ampule filled with medical fluid such as injection is usually subjected to a sterilizing treatment during its manufacturing process and the microwave sterilization is well known as one example of said sterilizing treatment.

Such microwave sterilization relies on a phenomenon that medical fluid filling the ampule is heated upon certainly absorption of the microwave energy and is certainly advantageous in that desired sterilization is achieved in a very short time. However, such well known method of microwave sterilization is disadvantageous in that a temperature within the ampule is apt to become uneven, a trend of temperature rise (temperature rise curve) depends upon a particular type of medical fluid filling the ampule and a gaseous phase within the ampule is apt to be insufficiently heated, resulting in imperfect sterilization.

These Countermeasures are disclosed, for example, by Japanese Disclosure Gazette No. 1973-61609 entitled "Apparatus for sterilization of sealed ampule filled with medical fluid Japanese Disclosure Gazette No. 1973-59976 entitled "Apparatus for sterilization of medical fluid within ampule; and Japanese Disclosure Gazette No. 1979-34590 entitled Method for sterilization within container".

The microwave absorptivity generally depends upon various factors such as conductivity of medical fluid and, in addition, size of the ampule as well as quantity of medical fluid filling this may vary. Therefore, microwave irradiation under the same conditions without proper consideration of these factors might disadvantageously lead to different degrees of temperature rise, depending upon the type of medical fluid and the size of the ampule.

In view of this, none of the well known techniques as set forth above has effective adaptability for the temperature rise characteristic varying depending upon the type of medical fluid and thus achieves uniform sterilizing effect.

More specifically, the apparatus in accordance with the above-mentioned Japanese Disclosure Gazette No. 1973-61609 aims at the uniform temperature rise within the ampule by rotating the ampule which is being held at an appropriate angle with respect to the vertical as the ampule is irradiated with the microwaves. However, the intended temperature rise uniformity is unacceptably limited in spite of a considerably complicated mechanism required for this purpose.

The apparatus well known from the above-mentioned Japanese Disclosure Gazette No. 1973-59976 is claimed to minimize a differential temperature within the ampule by irradiating the lower portion of the ampule held upright with the microwaves. However, this apparatus also has various problems remaining unsolved due to a simple manner of microwave irradiation without any subtleness being worthy of notice. Namely, a low heating efficiency requires a correspondingly long heating furnace as well as a high microwave capacity and makes it impossible to achieve an efficient sterilization.

The known process and apparatus are especially disadvantageous because the dose of exposure is controlled only by adjusting the depth of insertion of the ampules into the slot.

Moreover, the microwave irradiation performed in such manner causes substantially only the liquid phase formed by medical fluid within the ampule to be heated and the unfilled space within the same ampule remains at a lower temperature even after medical fluid has attained a temperature enough high to be sterilized. In other words, germs on the inner wall of said unfilled space are left unsterilized.

Finally, the above-mentioned Japanese Disclosure Gazette No. 1979-34590 proposes a countermeasure of inverting the ampule. However, the mechanism for this purpose is considerably complicated and it is practically difficult for such mechanism to realize continuous processes of sterilization.

Additionally, if conditions of the microwave irra diation are changed according to the type of medical fluid, the velocity of sterilizing process should necessarily vary and accordingly the velocity of medical fluid filling process must be changed. This often results in a repression of production rate.

### SUMMARY OF THE INVENTION

In view of the drawbacks as have been indicated with respect to the prior art, an object of the present invention is to provide method and apparatus adapted to sterilize the ampule, using a relatively simplified mechanism, with a heating efficiency adjustable for particularities depending upon the case, for example, conductivity of particular medical fluid and a size of particular ampule.

Such object is achieved, according to the present invention, by a method for ampule sterilization utilizing microwaves, in which only a lower portion of each ampule is inserted into an irradiation furnace provided in communication with a rectangular waveguide through a slot formed in top wall of said irradiation furnace and sterilization occurs during movement of the ampule along said slot, said method being characterized by a step of adjusting a depth of said irradiation furnace and a distance between the ampule and a surface of said slot and thereby controlling an exposure dose of said ampule to the microwaves.

Said objects is also achieved, according to the present invention, by an apparatus for ampule sterilization utilizing microwaves including an irradiation furnace provided in communication with a rectangular waveguide and formed in top wall with a slot and conveyor means adapted to move, along said slot, each ampule having only a lower portion thereof inserted into said irradiation furnace through said slot, said apparatus being characterized by that a depth of said irradiation furnace and a distance between the ampule and a surface of said slot is adjustable.

In view of a fact that the unfilled space within ampule is usually prevented from being adequately heated by the microwaves, these method and apparatus may be constructed so that the ampule is subjected to heating by hot blast during movement thereof along the slot.

The rectangular waveguide specified by JIS standards is too large to be used with the irradiation furnace to sterilize the ampules and, therefore, the cross-sectional area of the irradiation furnace is dimensioned according to the present invention so as to be smaller than the cross-sectional area of the rectangular waveguide.

Another object of the present invention is to provide a method allowing an ampule to attain a peak temperature at predetermined point during a process of ampule sterilization.

This object is achieved, in accordance with the present invention, by method or apparatus adapted to perform the microwave irradiation in a direction reverse to the direction in which the ampules move.

In spite of a relatively simplified mechanism involved, the present invention is extremely useful in practice in that medical fluid filling the ampule can be uniformly sterilized within the irradiation furnace having a high electric field strength by generating a connection in medical fluid filling the ampule and the ampules of different types and sizes can be flexibly treated because the heating efficiency for sterilization can be adjusted for various factors such as size and shape of the ampule as well as type of medical fluid filling the ampule.

Furthermore, the present invention permits the ampule sterilization to be carried out, by a relatively simplified mechanism, with the heating efficiency being adjustable for the particular medical fluid conductivity and ampule size, and thereby permits an efficient sterilizing treatment to be achieved with a sufficient compatibility with a working velocity of the medical fluid filling apparatus.

### BRIEF DESCRIPTION OF THE DRAWINGS

The above and other objects of the invention will be seen by reference to the description taken in connection with the accompanying drawings, in which:
Fig. 1 is a sectional side view showing an embodiment of the sterilizing apparatus constructed in accordance with the present invention;
Fig. 2 is a plane view showing the embodiment of Fig.1;

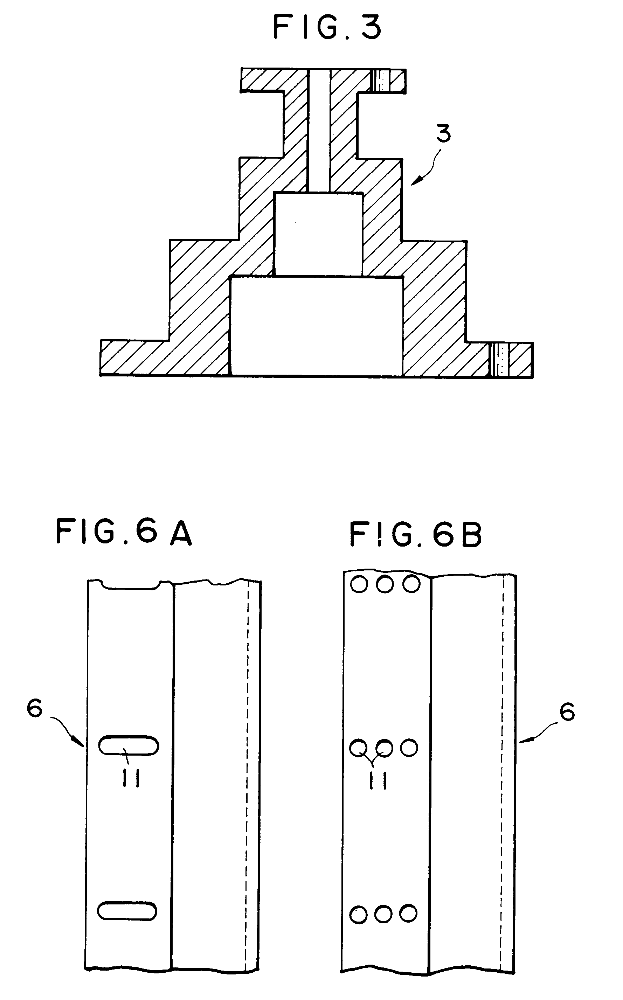

Fig. 3 is a sectional view showing an embodiment in which the cross-sectional area of the rectangular waveguide has been reduced relative to that in Fig.1 to define the irradiation furnace;
Fig.4 is a perspective view showing, partially in a section, a part of the irradiation furnace;
Figs. 5(A) through 5(C) are respectively fragmentary vertical sectional views illustrating an embodiment in which a depth(9) of the irradiation furnace (42) and /or a distance (10) between the ampule(30) moving along the slot(4) and a surface (4′) of the slot (4) are adjustable;
Figs. 6(A) and 6(B) are plane views respectively showing variants of a member defining the top of the irradiation furnace;
Fig. 7 is a vertical sectional views showing a heater section in Fig. 1, more in detail;
Fig. 8 is a vertical sectional view showing conveyor means in Fig. 2, more in detail;
Fig. 9 is a plane view showing a backet in Fig. 8, more in detail;
Fig. 10 is a graphic diagram illustrating how the temperature rise curve will be different between when the microwave irradiation occurs in the forward direction with respect to the direction in which the ampules is moved and when said irradiation occurs in the reverse direction with respect to the direction in which the ampule is moved; and
Figs. 11(A) through 11(C) are graphic diagrams illustrating the temperature rise curve of medical fluid versus the depth of the irradiation furnace and the distance between the ampule and the slot surface.

### DETAILED DESCRIPTION OF THE INVENTION

The present invention will be described, by way of example, in reference with the accompanying drawings.

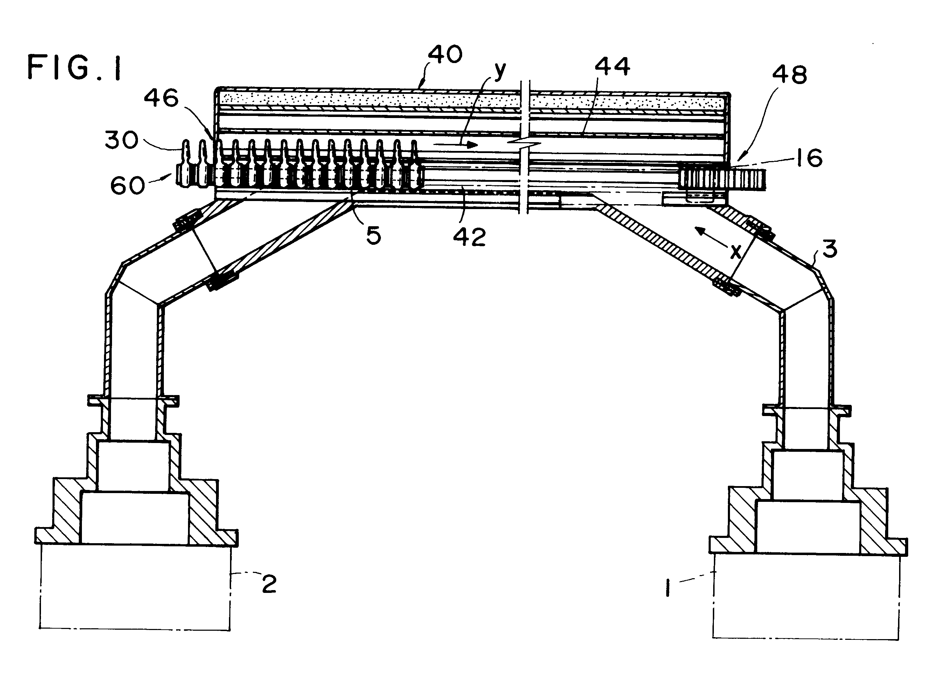

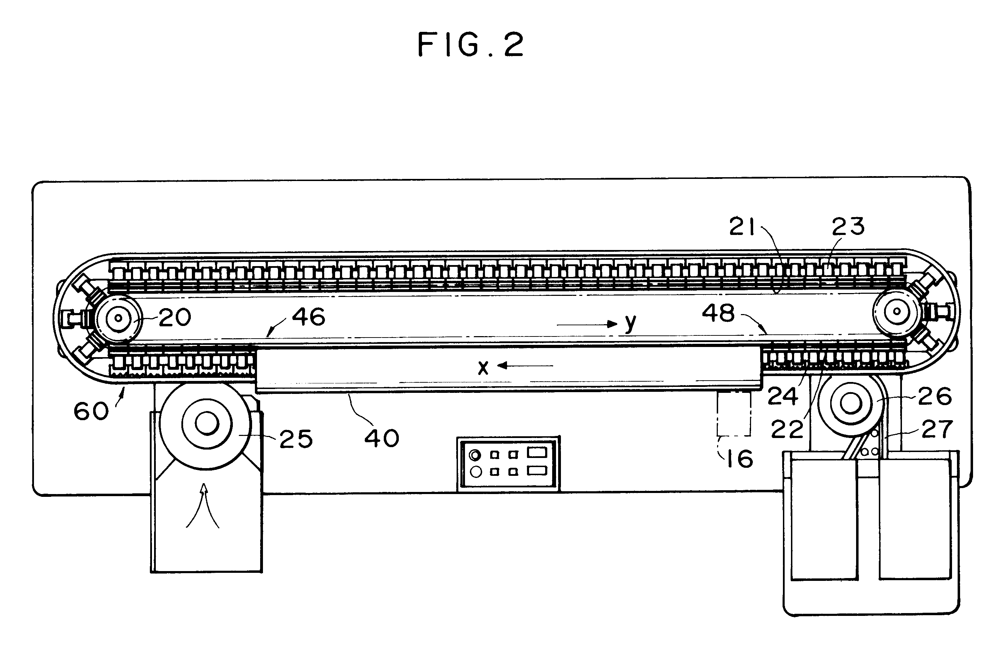

Figs. 1 and 2 illustrate a sterilizing apparatus constructed in accordance with the present invention, which comprises, as important components, a heater section (40) and conveyor means (60) adapted to transport ampules (30) through said heater section (40).

First, the heater section (40) will be discussed with reference to Fig. 1.

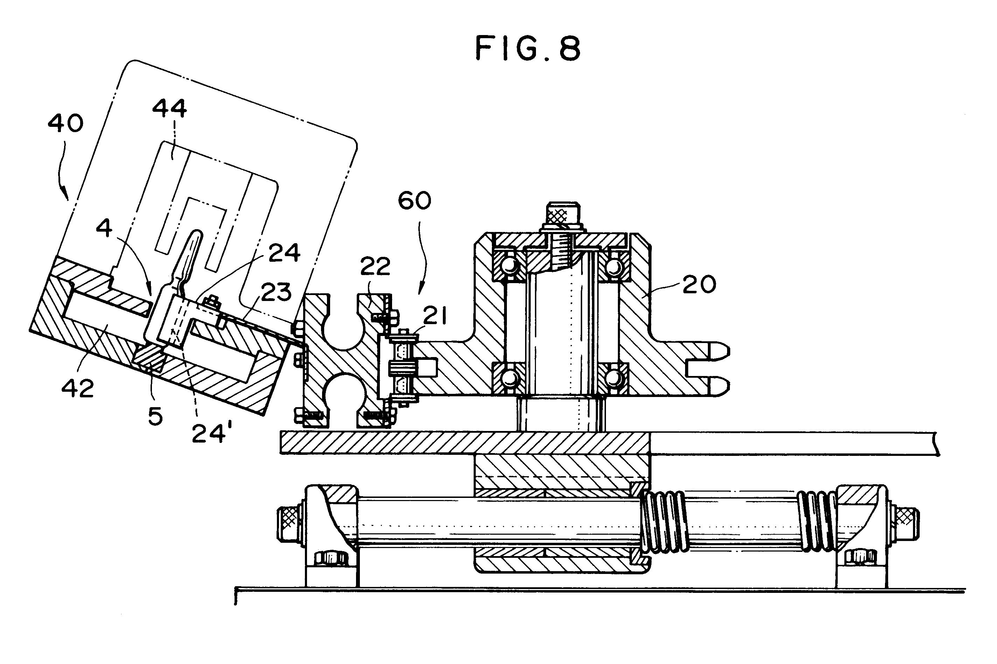

The heater section(40) comprises an irradiation furnace (42) formed by reducing a cross-sectional area of a rectangular waveguide (3) provided in communication with a microwave generator (1), on one side, and with a microwaves absorber(2), on the other side, to propagate microwaves from the former to the latter in a direction as indicated by an arrow(x), and a hot blast furnace (44) covering a top of said irradiation furnace (42) from above. It should be understood here that heater section (40) is disposed to be inclined inwardly with respect to the conveyor means (60) at some degrees (in order of 20 degree ) from the vertical, as seen in Fig. 8.

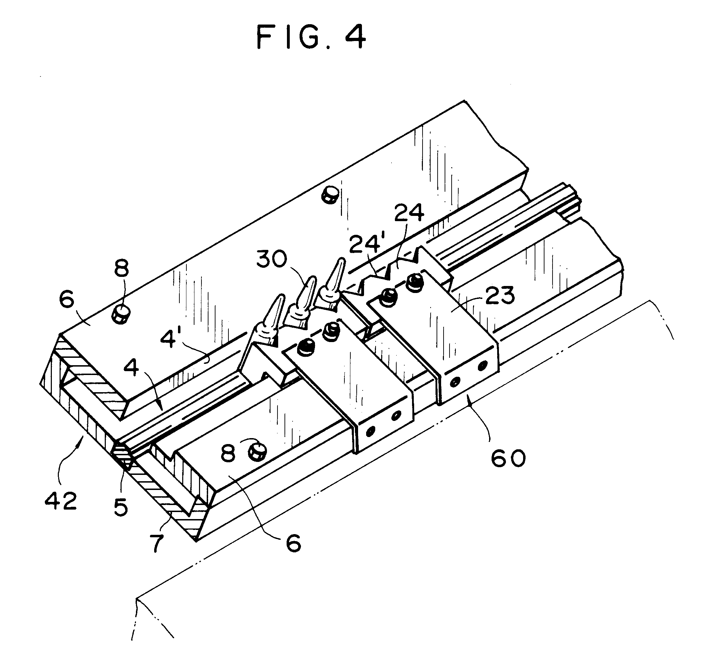

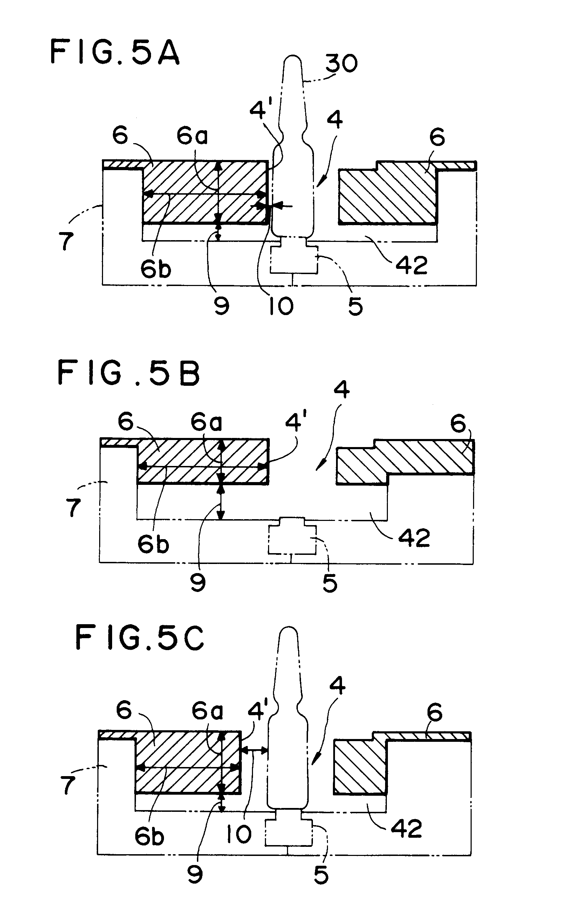

As will be apparent from Fig. 4, the irradiation furnace (42) is provided in its upper wall with a slot (4) extending longitudinally of the furnace so that the ampules (30) having only lower portions thereof inserted through the slot (4) into the irradiation furnace (42) may be transported along said slot (4) by the conveyor means (60) which will be described more in detail later. A guide (5) used to support bottoms of the ampules (30) is exchangeable in accordance with a particular height of said ampules(30). A member (6) forming the upper wall of the irradiation furnace (42) is also exchangeable and adapted to be secured to a furnace body (7) by bolts (8). Specifically, the member (6) of selected thickness (6a) and width (6b) may be exchangeably mounted on the furnace body (7) to adjust a depth (9) of the irradiation furnace (42) as well as a distance (10) between the respective ampules(30) and a surface (4′) of the slot (4), as will be apparent from Figs. 5(A) through 5 (C).

Fig. 5(A) shows a case in which the distance (10) between the respective ampules (30) and the surface (4′) of the slot (4) has been adjusted to be relatively narrow, Fig. 5(B) shows another case in which the depth (9) of the irradiation furnace(42) is larger than that in the case of Fig. 5(A), and Fig. 5(C) shows still another case in which the said distance (10) is larger than that in the case of Fig.5(A).

A cross-sectional area of the irradiation furnace (42) may be dimensioned so as to be smaller than that of the rectangular waveguide(3) to improve a power flux density within the irradiation furnace (42) and correspondingly to improve a heating efficiency. Thus, the effective length of the irradiation furnace can be shortened, making it possible to miniaturize the apparatus as a whole.

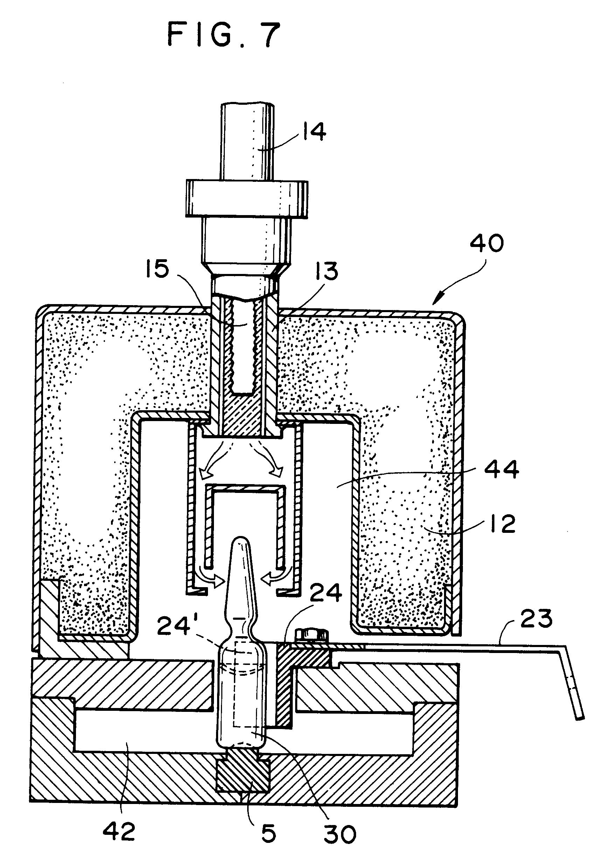

Fig. 6(A) shows a case in which the member(6) is provided with elongate through-holes(11) for the bolts (8) while Fig. 6(B) shows alternative case in which the member (6) is provided with a plurality of through-holes (11) for the bolts(8). In any case, it is possible with the single and same member to adjust the distance between the respective ampules (30) and the surface (4′) of the slot (4).

Referring to Fig. 7, the top of the irradiation furnace (42) is covered with the hot blast furnace (44) which is, in turn, isolated by a heat shielding wall (12) filled with suitable thermal insulating material from the exterior.

Reference numeral (13) designates a nozzle adapted to supply the hot blast furnace (44) with hot blast and compressed air supplied through a pipe (14) flows then through said nozzle (13) and a heater (15) disposed therein into the furnace(44) so as to heat upper portions of the respective ampules(30).

Referring again to Fig. 1 there is provided adjacent an outlet(48) of the heater section(40) constructed as has been mentioned a thermometer(16) such as an infrared radiation thermometer for remotely measuring a temperature of the ampules(30).

Now the conveyor means(60) will be explained.

Referring to Fig. 2, reference numerals (20), (20) designate a pair of sprockets comprising a driver sprocket adapted to be counterclockwise rotatably driven by an electromoter not shown and a follower sprocket.

These sprockets(20), (20) are operatively associated by an endless chain(21) which carries, in turn, conveyor bodies(22), as shown in Fig. 8, and plates(23) extending from the respective conveyor bodies(22) are provided on front ends thereof with buckets(24) to be received by the slot(4). Each backet(24) is inclined inwardly just as the heater section(40) is, so that the ampules(30) are also correspondingly inclined in the respective notches(24′) holding the respective ampules(30) with bottoms thereof bearing against the guide(5) in the course during which the ampules(30) are transported in a direction(y) as the pair of said sprockets(20), (20) are rotated.

Preferably, the buckets(24) are made of material such as fluorocarbon resin, having a low specific inductive capacity and a high heat resistance.

Each conveyor body(22) is formed in top and bottom with grooves, respectively, each having a cylindrical groove bottom. Although not shown here, along linear sections of the chain course, the conveyor bodies(22) are guided by rods extending through these grooves, respectively, and thereby these conveyor bodies(22) can transport the respective ampules(30) held in the respective notches(24′) of the individual buckets(24) with a high stability.

As shown in Fig. 2, there is provided adjacent the inlet(46) of heater section(40) feeder means(25) to feed the ampules(30) onto the conveyor means(60) while there is provided adjacent the outlet(48) of the heater section(40) take-out means(26) adapted to take the ampules(30) out from the conveyor means(60). Reference numeral(27) designates sorter means adapted to sort the ampules(30) taken out by the take-out means(26) into products of acceptable quantity and products of unacceptable quality.

With the embodiment of the present invention as has been described hereinabove, along the heater section(40), the ampules(30) being transported by the conveyor means(60) are irradiated only at the lower portions thereof inserted into the irradiation furnace(42) with the microwaves and, as a result, a temperature difference is generated in medical fluid filling each ampule(30) between upper and lower portions thereof. Such temperature difference produces a convection in said medical fluid and this convection is utilized to uniformly irradiate throughout said medical fluid filling the ampule(30) with the microwaves during movement thereof together with the conveyor means(60) along the heater section(40).

Concerning the direction in which the ampules(30) are irradiated with the microwaves in the irradiation furnace(42), such irradiation may occur in the same direction as the direction(y) in which the ampules(30) are transported or in the direction(x) reverse to the direction(y) in which the ampules(30) are transported. However, the microwave irradiation occurring in the direction(x) reverse to the direction(y) in which the ampules(30) are transported has been found to be significantly effective in temperature control for the ampules(30).

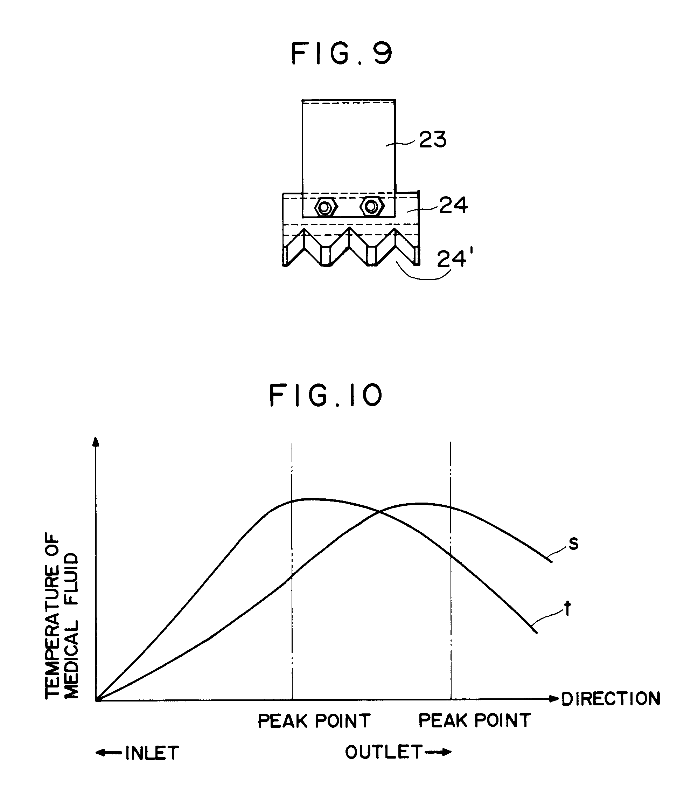

Fig. 10 plots how the temperature rise curve varies depending upon whether the microwave irradiation occurs in the same direction as the direction(y) in which the ampules(30) are transported or in the direction(x) reverse to said direction(y).

Specifically, when the microwave irradiation occurs in the direction(x) reverse to the direction(y) in which the ampules(30) are transported, no microwave absorption occurs at the outlet(48) of the irradiation furnace(42) and, as a result, the temperature ceases to rise, so that the temperature rise curve(s) exhibited when the irradiation occurs in the reverse direction attains to its peak as the ampule(30) reaches the outlet(48).

On the contrary, when the microwave irradiation occurs in the same direction as the direction(y) in which the ampules(30) are transported, the peak position on the temperature rise curve(t) exhibited by such irradiation occurring in the forward direction can not be constant, because the point along the irradiation furnace at which the microwave absorption ceases depends upon the particular type of medical fluid fill ing the ampule. Specifically, the particular type of medical fluid has a conductivity and therefore a temperature rise characteristic peculiar thereto. As an inevitable result, the peak point on the temperature rise curve(t) is variable. Accordingly, it is necessary to change the point along the irradiation furnace at which the peak temperature of medical fluid is to be measured, depending upon the type of medical fluid, and appropriate technical means to achieve this purpose is also required, so far as the microwave irradiation in the forward direction is employed.

In contrast with this, the microwave irradiation in the direction(x) reverse to the direction(y) in which the ampules(30) are transported assures that the temperature rise curve(s) find its peak point when the ampule(30) reaches the outlet(48) of the irradiation furnace (42). Therefore, the thermometer (16) may be disposed at the outlet (48) to precisely measure the peak temperature and thereby to determine a sterilizing effect through the temperature control.

Another important feature of the method according to the present invention lies in that the depth(9) of the irradiation furnace(42) and/or the distance(10) between the ampule(30) and the surface(4′) of the slot(4) may be adjusted to adapt exposure dose as well as strength of the microwaves for the variable factors such as size and shape of the ampule(30) and type of medical fluid.

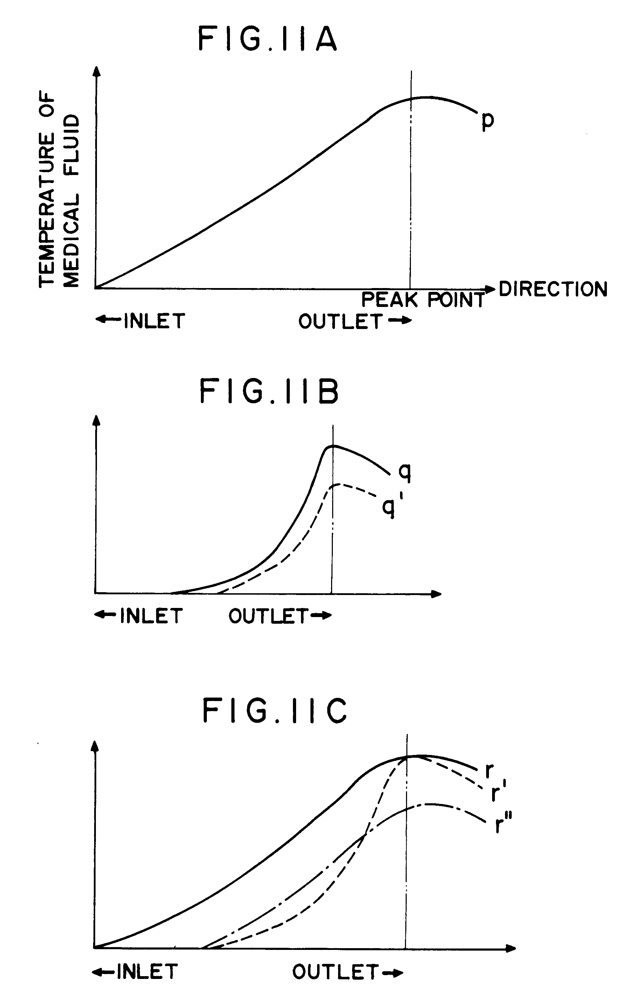

Now it will be considered, in reference with Fig. 11, how the temperature rise curve of medical fluid depends upon the depth(9) of the irradiation furnace(42) as well as the distance(10) between the ampule(30) and the surface(4′) of the slot(4). It should be noted here that Figs. 11(A) through 11(C) illustrate the case in which the microwave irradiation occurs in the direction(x) reverse to the direction(y) in which the ampules(30) are transported.

Fig. 11(A) illustrates a temperature rise curve(p) plotted in connection with medical fluid of the type that is poor in the microwave absorption efficiency. As shown, the curve(p) has a gentle slope between the inlet(46) and the outlet(48) of the irradiation furnace(42) and, therefore, requires no substantial compensation, but it would otherwise be impossible for such medical fluid which is extremely poor in the microwave absorption efficiency to obtain a sufficient peak temperature. In order to overcome this in convenience, in accordance with the present invention, the depth of the irradiation furnace is selectively changed to adjust a level to which the ampules are inserted through the slot into the irradiation furnace, as shown in Fig. 5(B), and thereby to adjust a microwave irradiation dose to be given to the lower portion of each ampule.

Namely, the depth of the irradiation furnace may be adjusted deeper to increase the surface area of each ampule and thereby to improve the microwave absorption efficiency of particular medical fluid filling this ampule. In this way, said medical fluid which would otherwise be poor in the microwave absorption efficiency can absorb a larger quantity of the microwave and thereby obtain a higher peak temperature.

Then, the case of medical fluid exhibiting a high absorption efficiency will be considered. Substantially whole microwave energy is absorbed by medical fluid as early as at a point adjacent the outlet(48) of the irradiation furnace(42) and substantially no microwave can be propagated to a point adjacent the inlet(42). A temperature rise curve(q) corresponding to such medical fluid exhibits a sharp rise at a point adjacent the outlet(48), as shown in Fig.11(B). Such a sharp temperature rise leads to an uneven temperature distribution in medical fluid, makes the sterilizing effect unreliable and sometimes causes the ampules of unacceptable quality. Nevertheless, when the microwave generator is controlled to lower the power level, there is a risk that the peak temperature is correspondingly lowered and the sterilizing effect unacceptably decreases, although a temperature rise curve(q′) of more gentle slope is obtained.

To avoid such risk, the distance(10) between the ampule(30) and the surface(4′) of the slot(4) may be changed to adjust the strength of the microwaves with which the ampule is irradiated and to make the temperature rise curve gentle. In Fig. 11(C), a temperature rise curve(r′) is plotted when the distance(10) between the ampule(30) and the surface(4′) of the slot(4) is shortened, as shown by Fig. 5(A), while a temperature rise curve(r˝) is plotted when said distance(10) is enlarged, as shown by Fig.5(C). In the former case, i.e., when said distance(10) is relatively narrow, the peak temperature is raised but the temperature rise curve becomes sharp.

In the latter case, i.e., when said distance(10) is relatively wide, on the contrary, the peak temperature is slightly lowered but the temperature rise curve is gentle. Accordingly, not only said temperature rise curve(r˝) may be obtained by enlarging the distance(10) but also the power level may be raised in order to obtain a temperature rise curve similar to the temperature rise curve(r) which is acceptably gentle and exhibits a desired peak.

The temperature rise curve established when the distance(10) has been adjustable enlarged, which presents a slightly lowered peak temperature but a more gentle slope in comparison to the case in which said distance(10) is relatively narrow, can be explained by the fact that a microwave amount absorbed by each ampule decreases and the microwaves are propagated to correspondingly larger number of the following ampules.

According to the present invention, as will be readily understood from the foregoing description, the depth(9) of the irradiation furnace(42) and/or the distance(10) between the respective ampules(30) and the surface(4′) of the slot(4) may be adjustable adapted for the variable factors such as size and shape of the ampules(30) as well as particular type of medical fluid filling these ampules(30) to obtain a temperature rise curve presenting a gentle slope and a desired peak temperature. Thus, not only a temperature rise within the ampule become gentle but also a convection within the ampule is gradually generated, advantageously resulting in that the temperature within each ampule becomes uniform and the ampules exhibiting any abnormal temperature rise becomes few.

Also in accordance with the present invention, upper portions of the respective ampules(30) may be heated by hot blast, as in the previously mentioned embodiment, to heat the unfilled space of each ampule and thereby to sterilize the inner wall of said unfilled space remaining not heated by the microwaves.

Furthermore, the cross-sectional area of the irradiation furnace(42) may be dimensioned so as to be smaller than that of the rectangular waveguide(3) in order that the power flux density within the irradiation furnace(42) is increased and the heating efficiency is correspondingly improved, permitting the irradiation furnace to be shortened and thereby permitting the apparatus as a whole to be miniaturized.

It should be understood that the diameter of the rectangular waveguide(3) may be stepwise reduced in the direction of microwave propagation to increase the power flux density and correspondingly to improve the heating efficiency.

It will be apparent from the foregoing description that, according to the present invention, the depth of the irradiation furnace may be selectively changed and thereby the vertical level to which the ampules are inserted through the slot into the irradiation furnace may be changed to adjust a microwave dose to be given to the lower portion of each ampule, on one hand, and the distance between the ampules and the a surface of the slot may be selectively changed to adjust a microwave intensity to which each ampule is exposed, on the other hand.

The feature of this invention that only the lower portion of the respective ampules inserted into the irradiation furnace are irradiated with the microwaves causes a significant differential temperature between the upper and lower portions of the respective ampules and thereby generates a convection within the ampules. Such convection permits the whole amount of medical fluid filling the respective ampules to be irradiated with the microwaves and thereby to be uniformly heat sterilized during movement of the ampules along the slot.

Furthermore, the upper portion of each ampule may be heated by hot blast in order to heat the unfilled space within the ampule and to sterilize the inner wall of such unfilled space.

Additionally, the cross-sectioinal area of the irradiation furnace may be reduced with respect to that of the rectangular waveguide to increase a power flux density of the heating efficiency. This allows, in turn, the irradiation furnace to be shortened.

Moreover, the microwave irradiation may be performed in the direction reverse to that in which the ampules are transported in order to assure that the ampules moving along the slot always cease to absorb the microwaves until the respective ampules reach the outlet of the irradiation furnace and thus exhibit the peak temperature at a constant point along the slot.

## Claims

1. A method for the sterilization of ampules (30) utilizing microwaves, said ampules (30) being exposed to a microwave field inside a tubular waveguide (6,6,7), in the course of which
(a) only the lower portion of each ampule (30) is inserted to a certain depth into the zone irradiated by the microwaves (irradiation furnace (42)) inside said waveguide (6,6,7),
(b) said irradiation furnace (42) is open through its top wall (6,6) through a slot (4) embracing said inserted ampules (30) during their transport through said waveguide (6,6,7),
(c) and the dose of exposure is controlled by adjusting the depth of insertion of the ampules into said slot (4),
characterized in that the dose of exposure is controlled in addition by adjusting the distance between the ampule (30) and a surface (4') of said slot (4).

2. Method as recited in claim 1, wherein the irradiation furnace (42) has a cross-sectional area smaller than that of a rectangular waveguide (3) which provides the microwave energy and to which it is connected.

3. Method as recited in claim 1 or 2, wherein the microwave irradiation travels in the direction reverse to-the direction in which the ampules (30) are transported.

4. Method as recited in claim 1, wherein an upper portion of the ampule is heated by hot blast during movement of said ampule along said slot (4).

5. Apparatus for the sterilization of ampules (30) utilizing microwaves, wherein said ampules (30) are exposed to a microwave field inside a tubular waveguide and
(a) only the lower portion of each ampule (30) is insertable to a certain depth into the zone irradiated by the microwaves (irradiation furnace (42)) inside said waveguide (6,6,7),
(b) said irradiation furnace (42) is open through its top wall (6,6) through a slot (4) embracing said inserted ampules (30) during their transport through said waveguide (6,6,7),
(c) and the dose of exposure is controllable by adjusting the depth of insertion of the ampules into said slot (4),
characterized in that the dose of exposure in addition is controllable by adjusting the distance between the ampule (30) and the surface (4') of said slot (4).

6. Apparatus as recited in claim 5, wherein member(s) (6,6) forming the top wall of the irradiation furnace (42) are exchangeable to adjust the depth (9) of the irradiation furnace (42) and the distance (10) between ampules (30) and an inner surface (4') of the slot (4).

7. Apparatus as recited in claims 5 or 6, wherein the irradiation furnace (42) has a cross-sectional area-smaller than that of the rectangular waveguide (3).

8. Apparatus as recited in claims 5 to 7, wherein the conveyor means (20, 21) is adapted to transport the ampules (30) in the direction reverse to that in which the microwave irradiation travels.

9. Apparatus as recited in claims 5 to 7, characterized in that a hot blast furnace (heater 15) is provided above the irradiation furnace (42).

10. Apparatus as recited in claims 5 to 9, wherein the conveyor means comprises a plurality of buckets (24) each having a plurality of notches (24') used to transport the associated ampules (30),
and wherein the respective ampules are engaged with the associated notches (24') in a plane inclined inwardly with respect to the vertical in order to assure that the respective ampules are engaged with said associated notches (24') in a stable manner.

## Patentansprüche

1. Verfahren zum Sterilisieren von Ampullen (30) mit Hilfe von Mikrowellen, bei dem die Ampullen (30) einem Mikrowellen-Feld innerhalb eines rohrförmigen Wellenleiters (6,6,7) ausgesetzt werden, wobei im Verfahren
(a) nur der untere Abschnitt jeder Ampulle (30) bis zu einer bestimmten Tiefe in die Zone innerhalb des Wellenleiters (6,6,7) eingesetzt wird, die von Mikrowellen bestrahlt wird [Bestrahlungsofen (42)],
(b) der Bestrahlungsofen (42) durch seine obere Wand (6,6) durch einen Schlitz (4) offen ist, der die eingesetzten Ampullen (30) während ihres Transportes durch den Wellenleiter (6,6,7) umfaßt,
(c) und die Bestrahlungsdosis durch Einstellung der Einsatztiefe der Ampullen in den Schlitz (4) gesteuert wird ,
dadurch gekennzeichnet, daß
die Bestrahlungsdosis zusätzlich durch Einstellung der Entfernung zwischen der Ampulle (30) und einer Fläche (4') des genannten Schlitzes (4') gesteuert wird.

2. Verfahren nach Anspruch 1, dadurch gekennzeichnet, daß der Bestrahlungsofen (42) einen Querschnitt hat, der kleiner ist als der eines rechteckigen Wellenleiters (3), der die Mikrowellenenergie zuführt und mit dem er verbunden ist.

3. Verfahren nach Anspruch 1 und 2, dadurch gekennzeichnet, daß die Mikrowellenstrahlung in einer Richtung sich ausbreitet, die entgegengesetzt der Ampullen-Transport-Richtung ist.

4. Verfahren nach Anspruch 1, dadurch gekennzeichnet, daß ein oberer Abschnitt der Ampulle durch ein Wärmegebläse während der Bewegung der Ampulle im Schlitz (4) erhitzt wird.

5. Vorrichtung zum Sterilisieren von Ampullen (30) unter Anwendung von Mikrowellen, in der die Ampullen (30) einem Mikrowellen-Feld innerhalb eines rohrförmigen Wellenleiters ausgesetzt werden und
(a) nur der untere Abschnitt jeder Ampulle (30) bis zu einer bestimmten Tiefe in die Zone innerhalb des Wellenleiters (6,6,7) einsetzbar ist, die von Mikrowellen bestrahlt wird
(b) der Bestrahlungsofen (42) durch seine obere Wand (6,6) durch einen Schlitz (4) offen ist, der die eingesetzten Ampulen (30) während ihres Transportes durch den Wellenleiter (6,6,7) umfaßt,
(c) und die Bestrahlungsdosis durch Einstellung der Einsatztiefe der Ampullen in den Schlitz (4) steuerbar ist,
dadurch gekennzeichnet, daß
die Bestrahlungsdosis zusätzlich durch Einstellung der Entfernung zwischen der Ampulle (30) und einer Oberfläche (4') des genannten Schlitzes (4) steuerbar ist.

6. Vorrichtung nach Anspruch 5, dadurch gekennzeichnet, daß der Teil bzw. die Teile (6,6), die die obere Wand des Bestrahlungsofens (42) bilden, auswechselbar sind, um die Tiefe (9) des Bestrahlungsofens (42) und die Entfernung (10) zwischen Ampullen (30) und innerer Oberfläche (4') des Schlitzes (4) einzustellen,

7. Vorrichtung nach Anspruch 5 oder 6, dadurch gekennzeichnet, daß der Bestrahlungsofen (42) einen Querschnitt hat, der kleiner ist als der des rechteckigen Wellenleiters (3).

8. Vorrichtung nach Anspruch 5 bis 7, dadurch gekennzeichnet, daß der Förderer (20, 21) zum Transport der Ampullen (30) in einer Richtung geeignet ist, die entgegengesetzt ist zuder, in der die Mikrowellen-Strahlung sich ausbreitet.

9. Vorrichtung nach Anspruch 5 bis 7, dadurch gekennzeichnet, daß ein Heizgebläse-Ofen (Erhitzer 15) oberhalb des Bestrahlungsofens (42) vorgesehen ist.

10. Vorrichtung nach Anspruch 5 bis 9, dadurch gekennzeichnet, daß der Förderer eine Vielzahl von Aufnahmevorrichtungen (24) umfaßt, deren jede eine Vielzahl von Nuten (24') zum Transport der entsprechenden Ampullen (30) besitzt,
wobei darin die jeweiligen Ampullen von den entsprechenden Nuten (24') in einer Ebene erfaßt sind, die in Bezug zur Vertikalen nach innen geneigt ist, um sicherzustellen, daß die jeweiligen Ampullen mit den betreffenden Nuten (24') stabil verbunden sind.

## Revendications

1. Procédé pour la stérilisation d'ampoules (30) utilisant des micro-ondes, lesdites ampoules (30) étant exposées dans un champ de micro-ondes à l'intérieur d'un guide d'ondes tubulaire (6,6,7), au cours duquel
(a) seule la partie inférieure de chaque ampoule (30) est insérée à une certaine profondeur dans la zone irradiée par les micro-ondes (four d'irradiation (42)) à l'intérieur dudit guide d'ondes (6,6,7),
(b) ledit four d'irradiation (42) est ouvert sur sa paroi supérieure (6,6) par une fente (4) contenant lesdites ampoules (30) insérées pendant leur transfert à travers ledit guide d'ondes (6,6,7),
(c) et le dosage de l'exposition est réglé par l'ajustement de la profondeur d'insertion des ampoules dans ladite fente (4),
caractérisé en ce que le dosage de l'exposition est réglé en complément par l'ajustement de la distance entre l'ampoule (30) et une face (4') de ladite fente (4).

2. Procédé tel que décrit dans la revendication 1, dans lequel le four d'irradiation (42) est pourvu d'une superficie de section transversale inférieure à celle d'un guide d'ondes rectangulaire (3) qui délivre l'énergie de micro-ondes et auquel il est raccordé.

3. Procédé tel que décrit dans la revendication 1 ou la revendication 2, dans lequel le rayonnement des micro-ondes se propage dans le sens opposé au sens de déplacement des ampoules (30).

4. Procédé tel que décrit dans la revendication 1, dans lequel une partie supérieure de l'ampoule est chauffée par jet chaud pendant le déplacement de ladite ampoule le long de ladite fente (4).

5. Appareil pour la stérilisation d'ampoules (30) utilisant des micro-ondes, dans lequel lesdites ampoules (30) sont exposées dans un champ de micro-ondes à l'intérieur d'un guide d'ondes tubulaire et
(a) seule la partie inférieure de chaque ampoule (30) peut être insérée à une certaine profondeur dans la zone irradiée par les micro-ondes (four d'irradiation (42)) à l'intérieur dudit guide d'ondes (6,6,7),
(b) ledit four d'irradiation (42) est ouvert sur sa paroi supérieure (6,6) par une fente (4)contenant lesdites ampoules (30) insérées pendant leur transfert à travers ledit guide d'ondes (6,6,7),
(c) et le dosage de l'exposition est réglé par l'ajustement de la profondeur d'insertion des ampoules dans ladite fente (4),
caractérisé en ce que le dosage de l'exposition est réglé en complément par l'ajustement de la distance entre l'ampoule (30) et une face (4')de ladite fente (4).

6. Appareil tel que décrit dans la revendication 5, dans lequel le(s) composant(s) (6,6) constituant la paroi supérieure du four d'irradiation (42) sont interchangeables dans le but d'ajuster la profondeur (9) du four d'irradiation (42) et la distance (10) entre les ampoules (30) et une face interne (4') de ladite fente (4).

7. Appareil tel que décrit dans les revendications 5 ou 6, dans lequel le four d'irradiation (42) est pourvu d'une superficie de section transversale inférieure à celle du guide d'ondes rectangulaire (3).

8. Appareil tel que décrit dans les revendication 5 à 7, dans lequel le moyen de transfert (20, 21) est conçu pour transférer les ampoules (30) dans le sens opposé à celui dans lequel se propage le rayonnement de micro-ondes.

9. Appareil tel que décrit dans les revendications 5 à 7, caractérisé en ce qu'un four à jet chaud (dispositif de chauffage 15) est disposé au-dessus du four d'irradiation (42).

10. Appareil tel que décrit dans les revendication 5 à 7, dans lequel le moyen de transfert comprend une pluralité de godets (24) chacun étant pourvu d'une pluralité d'encoches (24') utilisées pour transférer les ampoules (30) associées,
et dans lequel les ampoules respectives sont engagées dans les encoches associées (24') selon un plan incliné vers l'intérieur par rapport à la verticale, afin d'assurer que les ampoules respectives soient engagées de façon stable dans lesdites encoches (24').
